# EUROPEAN PATENT SPECIFICATION

(11) **EP 4 326 348 B1**
(45) Date of publication and mention of the grant of the patent: **11.12.2024**
(21) Application number: 22722756.8
(22) Date of filing: 13.04.2022
(51) Int. Cl.: A61L 2/10, F21V 11/02

(54) **DISINFECTING DEVICE HAVING IMPROVED EFFICIENCY AND SAFETY**
DESINFEKTIONSVORRICHTUNG MIT VERBESSERTER WIRKSAMKEIT UND SICHERHEIT
DISPOSITIF DE DÉSINFECTION AYANT UNE EFFICACITÉ ET UNE SÉCURITÉ AMÉLIORÉES

(30) Priority: 22.04.2021 EP 21169867
(43) Date of publication of application: 28.02.2024
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2022/059909
(87) International publication number: WO 2022/223405

(56) References cited:
- WO-A1-2023/285268
- WO-A2-2009/104119
- CN-A- 111 265 706
- CN-A- 111 282 012
- JP-A- H07 289 616
- KR-B1- 102 152 810

## Description

### TECHNICAL FIELD

The present invention relates to a disinfecting device having improved performance in term of efficiency and safety and being particularly suitable for upper air disinfection in a room.

### BACKGROUND

In view of the recent development in the world concerning the global pandemic, disinfection has become a topic of renewed interest as the demand for sterilization increases. One way of disinfecting involves the use of UV light. As a response to pathogenic outbreaks involving airborne microorganisms it would be beneficial to employ UV light for disinfecting air and objects at locations where the transmission of such microorganisms is believed to occur.

Disinfecting luminaires are used to flood spaces such as hospital rooms with UV-B (ultra-violet light of 280-315 nanometer (nm)) and UV-C (ultra-violet light of 100-280 nm) radiation for disinfection purposes. Such disinfecting luminaires require a relatively brief time, e.g. several minutes, to achieve adequate disinfection but require the room to be evacuated of people. Another type of disinfecting luminaire uses a fixed 405 nm violet light source to provide disinfection without evacuating people from the room. However, such luminaires may require hours to achieve adequate disinfection since violet light is less effective at killing pathogens compared to UV-B and UV-C radiation, and since the light dispersed over a wide area such that the irradiance level is relatively low.

Normally, disinfection by UV light sources is used under controlled conditions in areas where humans or animals are not present during ongoing disinfection, such as at surgery theaters or the like. However, the increased demand for germicidal activities may involve operating UV light sources in environments with human presence, thus introducing a risk for unintentional irradiation by UV light. Therefore, disinfecting light sources, in particular those involving UV light, should possess reliable safety features in order to avoid potential exposure of humans or animals to the harmful irradiation.

One possibility to eliminate or minimize such an exposure is using upper air disinfection. For this application, a light absorbing louver is used for controlling the spatial light distribution, such that the disinfecting light, in particular UV-C light, is distributed in the upper air of a room, i.e. the air arranged in vicinity of the ceiling, where people usually are not present. The louver removes harmful UV-C light emitted at higher angles and only allows predominantly horizontally arranged rays to be emitted. Such a light absorbing louver may thus be used in offices, malls, restaurants, as well as healthcare and education facilities, i.e. places being resided by humans and/or animals. However, upper air disinfection devices comprising a light absorbing louver suffers from the disadvantage of losing the majority of the UV-C light due to absorption by the louver, thus impairing the disinfecting efficiency of the device.

In view of the above, it is desired to obtain a disinfecting device having improved performance e.g. efficiency and safety compared to the solutions suggested by the prior art.

KR102152810B1 discloses an indoor sterilizing device capable of sterilizing air by collecting ultraviolet rays of an ultraviolet light source. Other examples are disclosed in CN111282012, CN111265706 and JPH07289616.

### SUMMARY

The invention is set out in the appended set of claims. The present invention thus provides such a disinfecting device. The disinfecting device of the present invention may be particularly suitable for disinfecting spaces with high level of activity, such as a waiting room in a hospital or a veterinary clinic, a public space such as a library, an office, a department store or the like, as well as public transportation means, such as busses or trains.

Throughout the description, directions will be addressed as "downward", "upward". In the context of the present invention, the term "downward direction" is to be understood as a direction aligned with a vector of gravitational acceleration. The vector of gravitational acceleration may be understood as being a gravitational acceleration vector of a celestial body, e.g. the Earth, on which the disinfecting device is arranged or located. It is intuitively understood that the term "upward direction" is the direction being opposite to the downward direction, i.e. a direction arranged 180° from the downward direction.

The disinfecting device comprises at least one light source arranged for, in operation, to emit a first light having a first UV wavelength range. The light source may be any light source configured to emit light having a high germicidal effect. In particular, the first light may have a major wavelength peak in the first UV wavelength range. In the context of the present invention, the UV-spectrum comprises any electromagnetic radiation with a wavelength from 10 nm to 400 nm. The first light may be omnidirectional, i.e. may be emitted 360° from the at least one light source.

The first UV wavelength range may be from 100 nm to 280 nm, i.e. the UV-C wavelength range. Further, the first UV wavelength range may be UV-B wavelength range (280 nm-315 nm), and UV-A wavelength range (315 nm-400 nm).

The light source may be a solid-state light source such as a light-emitting diode, LED, and/or a laser diode. Further, the light source may be a low pressure mercury plasma lamp or an excimer light source. The light source may comprise a plurality of LEDs each of which emits at least one of: UV-C radiation (100 nm-280 nm); UV-B radiation (280 nm-315 nm); UV-A radiation (315 nm-400 nm). By the term "plurality" in the context of the present invention is meant two or more.

The term "LED" as used in the context of the present invention implies any type of LED known in the art, such as inorganic LED(s), organic LED(s), polymer/polymeric LEDs, violet LEDs, blue LEDs, optically pumped phosphor coated LEDs, optically pumped nano-crystal LEDs. As used herein, the term "LED" can encompass a bare LED die arranged in a housing, which may be referred to as a LED package. When UV-C light is used, the LED may be mounted in a cavity covered in a non-contact manner by an emission window made from quartz/fused silica.

The plurality of LEDs may comprise at least 10 LEDs, preferably at least 20 LEDs, more preferably at least 30 LEDs.

The disinfecting device according to the present invention may comprise a plurality of light sources, each providing a first light. The wavelengths of the first light emitted from each light source may be same or different. For example, the disinfecting device may comprise three light sources, wherein the first one of the light sources emits light within the UV-C spectrum, the second one of the light sources emits light within the UV-B spectrum, and the third one of the light sources emits light within the UV-A spectrum. The various light sources might be used together or might be operated individually depending on the type of microbiological species that needs to be deactivated.

The disinfecting device of the present invention comprises at least one louver comprising a plurality of light absorbing structures having a first surface and a second surface being opposite to the first surface. The disinfecting device may be attached to a mounting surface, such as a ceiling or a wall. In such an embodiment, the first surface and the second surface are arranged substantially parallelly to the mounting surface. Further, the disinfecting device may be arranged such that the first and the second surface are substantially perpendicular to the mounting surface, e.g. in an embodiment when the mounting surface is a table, a floor, a shelf, or the like.

According to the invention, the plurality of light absorbing structures further has a proximal end being arranged adjacent to the at least one light source, and a distal end being arranged opposite to the proximal end. The plurality of light absorbing structures further has a longitudinal extension between the proximal end and the distal end, and a transversal extension being substantially perpendicular to the longitudinal extension. The light absorbing structure according to the present invention is arranged for absorbing a first portion of the first light. The first portion of the first light may preferably be at least 10%, more preferably at least 15%, most preferably at least 20% of the total amount of the first light. Further, the first portion may preferably be less than 35%, more preferably less than 30%, most preferably less than 25% of the total amount of the first light.

The plurality of light absorbing structures may be in the form of a plate, a cylinder, a triangular prism, a cuboid, a pentagonal prism, a hexagonal prism, or the like. Further, the plurality of light absorbing structures may be connected, and may form a single piece, honeycomb or grid structure.

The plurality of light absorbing structures may have a high aspect ratio, e.g. an aspect ratio of at least 2, preferably at least 3, more preferably at least 4, most preferably at least 5. By the term "aspect ratio" in the context of the present invention is understood the ratio between the longitudinal extension and the transversal extension of the plurality of light absorbing structures.

The disinfecting device according to the present invention further comprises at least one optical element arranged to collimate a part of the first light emitted from the light source towards the louver. In other words, the part of the first light that interacts with the at least one optical means is collimated, while the remaining part of the first light emitted towards the louver will not be collimated. Thus, the first light emitted by the at least one light source will be directed towards the light absorbing structure as a collimated beam comprising predominantly parallel rays of the first light. The collimated beam of first light is substantially parallel to the longitudinal extension of the plurality of light absorbing structures, for example a plurality of lamellae, and therefore will spread minimally as it propagates, thus minimizing dispersion with distance. As intuitively understood, a substantial portion of the first light will be transmitted through said louver without impinging on the light absorbing structure and emitted by the disinfecting device in a direction being substantially in plane with the longitudinal extension of the light absorbing structure in the case of lamellae, and in a direction being substantially in the direction of the longitudinal extension of the light absorbing structure in the case of honeycomb or grid structure. The optical element may be a lens, an array of lenses, a reflector, total internal reflection optical element (TIR optic), or any other type of collimator.

However, since a perfect collimation is prevented by diffraction, the beam of the first light collimated by the at least one optical element will comprise rays being non-parallel to the longitudinal extension of the plurality of light absorbing structures. Such rays will interact with the first and/or the second surface of the plurality of light absorbing structures. As mentioned above, a first portion of these rays of the first light will be absorbed by the plurality of light absorbing structures. The louver thus removes harmful UV-C light emitted at higher angles and only allows predominantly horizontally arranged rays of first light to be emitted , i.e. the third portion of the first light has a relatively narrow third spatial light distribution defined as full-width-half-max FWHM3, with FWHM3 being 5° or less, preferably 4° or less, such as 3° or less . The first light as emitted from the light source has a first spatial light distribution with a FWHM1, the first light which is directed via the at least one optical element to the louver has a second spatial light distribution FWHM2, wherein preferably FWHM1>FWHM2>FWHM3. The second light has a fourth spatial light distribution FWHM4, wherein preferably FWHM4 > FWHM3, and more preferably FWHM4 > FWHM2. In embodiments FWHM3 <= 0.5* FWHM2. The spatial light distributions indicated above may relate to the spatial light distributions measured in a vertical manner (with respect to the direction of gravity) onto the light exit window. The spatial light distributions exiting the light exit in a horizontal manner are typically less collimated. Particularly if parallel arranged staked plates are used.

In order to increase efficiency and safety of the disinfecting device according to the present invention, the plurality of light absorbing structures comprises at least one light converting element arranged for converting a second portion of the first light into a second light having a second wavelength range comprising longer wavelengths than the first UV wavelength range. The second portion of the first light may preferably be at least 10%, more preferably at least 15%, most preferably at least 20% of the total amount of the first light. Further, the second portion of the first light may preferably be less than 35%, more preferably less than 30%, most preferably less 25% of the total amount of the first light. It must be noted that the second portion of the first light is different from the first portion of the first light.

In other words, the portion of the first light consisting of rays of the first light being non-parallel to the longitudinal extension of the light absorbing structure comprises a first portion and a second portion, wherein the first portion is absorbed by the plurality of light absorbing structures, and the second portion is converted into a second light having a second wavelength range comprising longer wavelengths than the first UV wavelength range. In particular, the second light may have a major wavelength peak in the second wavelength range.

As the result, the light emitted by the disinfecting device of the present invention will comprise the first light having a first UV wavelength range, and a second light having a second wavelength range comprising longer wavelengths than the first wavelength range.

According to the present invention, the efficiency as well as safety of the disinfecting device is increased, since the potentially harmful first light, in particular UV-C light, will be prevented from exiting the disinfecting device at undesired angles, thus minimizing the risk of unintentional exposure of humans and/or animals to damaging irradiation. Instead, a portion of the dispersed first light will be converted into a less harmful second light, that may be UV-A light, UV-B light, visible light, or combination thereof. UV-A and/or UV-B light are less damaging compared to the UV-C light and may be used for disinfection purposes at wider angles. The visible light may be used for alerting the user that the disinfection device is operating. Alternatively, or additionally the visible light may be used for general illumination.

It should be noted that the second wavelength range comprises longer wavelengths than the first UV wavelength range. Thus, if the first UV wavelength range is a UV-C wavelength range, the second wavelength range may be UV-A wavelength range, UV-B wavelength range, visible wavelength range or combination thereof. If the if the first UV wavelength range is a UV-B wavelength range, UV-A wavelength range or combination thereof, the second wavelength range may be a visible wavelength range.

In particular, the second wavelength range may be from 280 nm to 780 nm, in particular from 400 nm to 780 nm.

The following combinations of the first UV wavelength range and the second wavelength range are particularly preferred according to the present invention:
the first UV wavelength range is from 100 nm to 280 nm, and the second wavelength range is from 280 nm to 380 nm;
the first UV wavelength range is from 100 nm to 280 nm, and the second wavelength range is from 280 nm to 400 nm;
the first UV wavelength range is from 100 nm to 280 nm, and the second wavelength range is from 280 nm to 420 nm;
the first UV wavelength range is from 100 nm to 280 nm, and wherein said second wavelength range is from 400 nm to 780 nm;
the first UV wavelength range is from 100 nm to 280 nm, and wherein said second wavelength range is from 280 nm to 780 nm.

According to the present invention, a third portion of the first light, i.e. the portion of the first light that has neither been absorbed nor converted, is exiting said disinfection device. The third portion of the first light may preferably be at least 20%, more preferably at least 30%, most preferably at least 35% of the total amount of the first light. Further, the third portion of the first light may preferably be less than 80%, more preferably less than 70%, most preferably less than 60% of the total amount of the first light. It should be noted that the third portion of the first light is different from the first portion and the second portion of the first light.

The light converting element comprises a light converting material. The light converting material converts the first UV light to the second light being UV light and/or visible light.

For example, UV-C light may be converted to UV-B, UV-A and/or visible light. The visible light may be white light or colored light (e.g. violet, blue, green, or red light). The white light may have a color temperature in the range from 2000 to 8000 K. The white light may have a CRI of at least 60, preferably at least 80.

According to the invention, the light converting element comprises a light converting material arranged in a matrix material such as a polymer matrix. Such a matrix may be a silicon matrix. The matrix material is light transmissive to the first light to increase the amount of conversion by the light converting element. The light converting material may be a phosphor. The at least one light converting element may be in the form of a coating and/or a layer.

The plurality of light absorbing structures may comprise a plurality of light converting elements, that may be arranged on the first surface of the light absorbing structure or on the second surface of the light absorbing structure. Alternatively, the plurality of the light converting element may be arranged on both the first and the second surfaces of the light absorbing structure.

The plurality of the light converting elements may be evenly or randomly distributed across the first and/or the second surface of the light absorbing structure. Each of the plurality of the light converting elements may convert the first light into a second light having a major wavelength peak in a second wavelength range being same as or different from the second wavelength range of the second light originating from the other light converting elements. For instance, the light absorbing structure of the present invention may comprise a first group of light converting elements for converting the first light into a second light within UV-A and UV-B spectrum, and a second group of light converting elements for converting the first light into a second light within the visible spectrum. The light emitted by such a disinfecting device will thus comprise UV-C light originating from the light source, UV-A and UV-B light originating from the first group of the light converting elements, and visible light originating from the second group of the light converting elements.

In order to improve safety of the disinfecting device according to the present invention, the light absorbing structure may comprise at least one wedge-shaped element being arranged on the first surface and/or the second surface of the light absorbing structure. By the term "wedge-shaped" in the context of the present invention is understood an element having a pointed edge at one end and a wide edge at the other. When the at least one wedge-shaped is present, it is arranged such that the wide edge is substantially perpendicular to the first and/or the second surface of the light absorbing structure, and such that the wide edge faces towards the at least one light source.

The at least one wedge-shaped element may comprise the light converting element. It is conceivable that the at least one light converting element is wedge-shaped. It is further conceivable that the light absorbing structure comprises at least one wedge-shaped element being arranged on the first surface and/or the second surface of the light absorbing structure. Such a wedge-shaped element may thus be an integral or an additional part of the light absorbing structure. The wedge-shaped element may be made of the same or different material as the light absorbing structure. In such an embodiment, the at least one light converting element may be arranged on the at least one wedge-shaped element. Preferably, the light converting element may be arranged as a coating on the wedge-shaped element.

In order to improve safety of the disinfecting device even further, the at least one light converting element may be arranged on a portion of the first surface and/or the second surface of the light absorbing structure. In particular, the area of the portion of the first surface and/or the second surface comprising the at least one light converting element may be less than 30%, preferably less than 25%, more preferably less than 22%, most preferably less than 20% of the total area of the first surface and/or the second surface. Further, the area of the portion of the first surface and/or the second surface comprising the at least one light converting element may be greater than 1%, preferably greater than 5%, more preferably greater than 8%, most preferably greater than 10% of the total area of the first surface and/or the second surface.

According to an embodiment of the present invention, the portion of the first surface and/or the second surface comprising the at least one light converting element may be arranged at the distal end of the light absorbing structure. In such an embodiment, visibility and efficiency of the disinfecting device is improved.

The at least one louver of the disinfecting device according of the present invention may comprise a plurality of light absorbing structures. The shape of each light absorbing structure may be same as or different from the shape of the other light absorbing structures. In particular, the plurality of light absorbing structures may comprise N light absorbing structures, wherein N is an integer being equal to or greater than 5, preferably equal to or greater than 7, more preferably equal to or greater than 8, even more preferably equal to or greater than 10, most preferably equal to or greater than 12. In a particularly preferred embodiment, the plurality of light absorbing structures is in the form of N substantially parallelly arranged plates.

According to an embodiment of the present invention, at least 5 light absorbing structures, preferably at least 8 light absorbing structures, more preferably at least 10 light absorbing structures comprise at least one light converting element being arranged on each of the first and the second surface.

In order to guarantee safety, the at least one light converting element may be arranged on K light absorbing structures, wherein K is an integer having a value from 1 to N-1. It is conceivable that at least 50%, preferably at least 80%, more preferably at least 90% of N light absorbing structures comprise at least one light converting element being arranged on each of the first and the second surface. In a particularly preferred embodiment, only the outmost light absorbing structures comprise at least one light converting element. By the term "outmost" is meant the light absorbing structures having at least one surface facing the ambient.

The disinfecting device may further comprise a housing comprising a light exit window. In the context of the invention a light exit window is to be interpreted as any area, volume, or material which allow light to pass through it. The housing may be a hermetic housing. The at least one light source, the at least one louver and the at least one optical element may be arranged inside the housing. The housing may have any geometrical shape. The housing may be formed as a cuboid, where at least one face of the cuboid may act as a light exit window from where light can be emitted. The housing may further comprise an attachment surface arranged to be positioned on the surface to which the housing is to be attached, such as a ceiling, a floor, a wall, a table, or another suitable surface in a room.

The disinfecting device may further comprise a UV light sensor arranged for determining the intensity of output UV light and its distribution within the room. This should not be considered as excluding other sensor types as presented in this disclosure.

The disinfecting device according to an embodiment of the present invention may be a luminaire having a mounting means for mounting the luminaire to a mounting surface. As mentioned above, when the mounting surface is a wall or a ceiling, the longitudinal extension of the plurality of light absorbing structures will be substantially parallel to the mounting surface. When the disinfecting device in the form of a luminaire is mounted on a wall, the light exiting the disinfecting device shall be directed upwards towards the ceiling of the room in order to avoid accidental exposure of a subject to the harmful UV light. The disinfecting device may be configured to suspend from a ceiling of a room by a suspension arrangement or may be attached to the ceiling.

Alternatively, the mounting surface may be the floor or a piece of furniture, such as a tabletop, a shelf, or the like. In such an embodiment, the longitudinal extension of the light absorbing structure will be substantially perpendicular to the mounting surface. Even in this case, the light exiting the disinfecting device will be directed upwards towards the ceiling of the room.

Finally, a plurality of disinfecting devices may be arranged at different locations within the same room in order to increase disinfection rate and capacity.

Considering the above, the present invention provides a safe and efficient disinfecting device. The disinfecting device provides high safety level, allowing ongoing disinfection even when humans and/or animals are present in the area that is being disinfected. Moreover, the safety level of the disinfecting device allows positioning the device substantially anywhere in the room, such as a wall or a table, which in turn provides an efficient disinfection.

### BRIEF DESCRIPTION OF THE DRAWINGS

Embodiments of the invention will now be described by way of example with reference to the accompanying drawings, of which:
Fig. 1a illustrates a cross-section of a disinfecting device according to the present invention;
Fig. 1b shows a top view of the disinfecting device depicted in Fig. 1a;
Figs. 2-4 depict various embodiments of the disinfecting device;
Figs. 5a and 5b illustrate wedge-shaped light converting elements.

All the figures are schematic, not necessarily to scale, and generally only show parts which are necessary in order to elucidate embodiments of the present invention, wherein other parts may be omitted or merely suggested.

### DETAILED DESCRIPTION

The present invention will now be described hereinafter with reference to the accompanying drawings, in which exemplifying embodiments of the present invention are shown. The present invention may, however, be embodied in many different forms and should not be construed as limited to the embodiments of the present invention set forth herein; rather, these embodiments of the present invention are provided by way of example so that this disclosure will convey the scope of the claims to those skilled in the art. In the drawings, identical or similar reference numerals denote the same or similar components having a same or similar function, unless specifically stated otherwise.

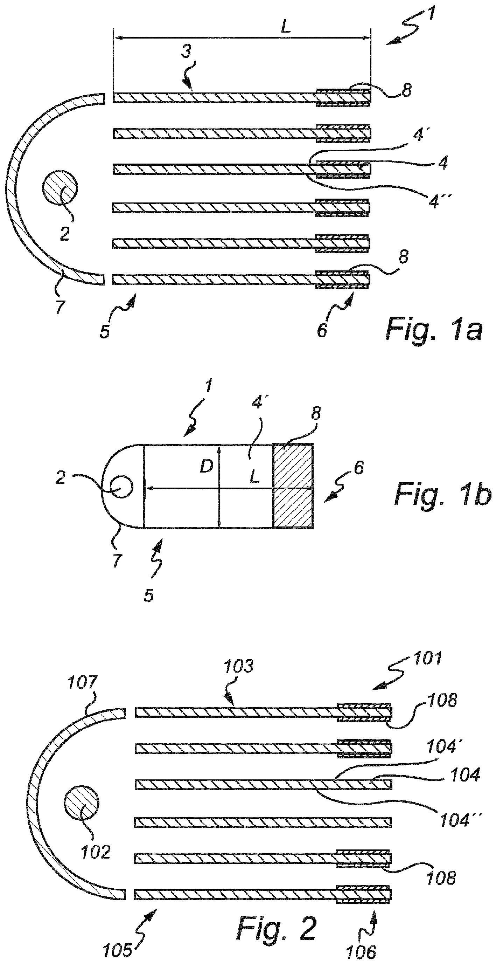

Figs. 1a and 1b illustrate a cross-section view and a top view, respectively, of the disinfecting device 1 according to the present invention. The disinfecting device 1 comprises one light source 2 arranged for, in operation, to emit a first light having a first UV wavelength range. The disinfecting device 1 further comprises a louver 3 comprising six light absorbing structures 4 each having a first surface 4' and a second surface 4" being opposite to the first surface 4'.

The light absorbing structures 4 further has a proximal end 5 being arranged adjacent to the light source 2, and a distal end 6 being arranged opposite to the proximal end 5. The light absorbing structures 4 further have a longitudinal extension L between the proximal end 5 and the distal end 6, and a transversal extension D being substantially perpendicular to the longitudinal extension L.

As may be seen in Figs. 1a and 1b, the light absorbing structures 4 are in the form of parallelly arranged stacked plates.

The light absorbing structures 4 have a high aspect ratio, e.g. an aspect ratio of at least 2, preferably at least 3, more preferably at least 4, most preferably at least 5. As mentioned above, by the term "aspect ratio" in the context of the present invention is understood the ratio between the longitudinal extension L and the transversal extension D of the light absorbing structure 4.

The disinfecting device 1 according to the present invention further comprises an optical element 7 arranged to collimate a part of the first light emitted from the light source 2 towards the louver 3. Thus, the first light emitted by the light source 2 will be directed towards the light absorbing structures 4 as a collimated beam comprising predominantly parallel rays of the first light. The collimated beam of first light is substantially in plane with the longitudinal extension L of the light absorbing structures 4. The optical element 7 is a reflector.

The light absorbing structures 4 comprise a plurality of light converting elements 8 arranged for converting a second portion of the first light into a second light having a second wavelength range comprising longer wavelengths than the first UV wavelength range.

As mentioned above, the portion of the first light consisting of rays of the first light being non-parallel to the longitudinal extension L of the light absorbing structures 4 comprises a first portion and a second portion, wherein the first portion is absorbed by the light absorbing structures 4, and the second portion is converted into a second light having a second wavelength range comprising longer wavelengths than the first UV wavelength range by means of the light converting elements 8.

As may be seen in Figs. 1a and 1b, each of the light absorbing structures 4 comprises a plurality of light converting elements 8, being arranged on a portion positioned at the distal end 6 of both the first and the second surfaces 4', 4" of the light absorbing structures 4. As mentioned above, by arranging the light converting elements 8 in a manner shown in Figs. 1a and 1b, visibility and efficiency of the disinfecting device 1 is improved. A third portion of the first light will exit the disinfecting device 1.

The embodiment depicted in Fig. 2 is similar to the embodiment shown in Figs. 1a and 1b, with the difference that the light converting elements 108 are arranged on four out of six light absorbing structures 104. As may be seen in Fig. 2, the light converting elements 108 are arranged on a portion positioned at the distal end 106 of both the first and the second surfaces 104', 104" of the two outmost light absorbing structures 104 on each side of the louver 103. In such an embodiment, safety of the disinfecting device 101 is improved.

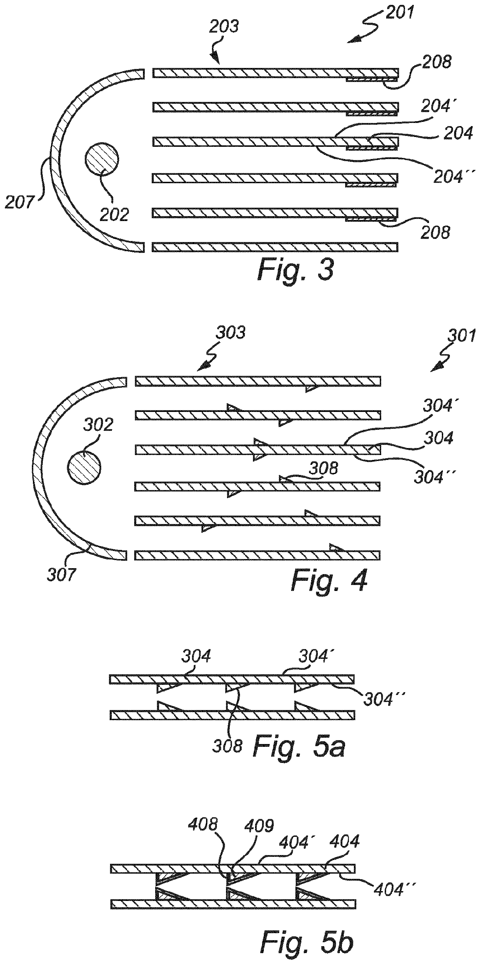

Fig. 3 illustrates yet another embodiment of the disinfecting device 201, wherein the light converting elements 208 are arranged on a portion positioned at the distal end 206 of the second surfaces 204" of the light absorbing structures 204. In such an embodiment, both visibility and safety of the disinfecting device 201 is improved.

Turning the attention to Fig. 4, a disinfecting device 301 is depicted, wherein the light converting elements 308 are wedge-shaped. As mentioned above, the term "wedge-shaped" in the context of the present invention means an element having a pointed edge at one end and a wide edge at the other. As may be seen in Fig. 4, the wedge-shaped light converting elements 308 are arranged such that the wide edges are substantially perpendicular to the first and/or the second surface 304', 304" of the light absorbing structures 304, and such that the wide edges face towards the light source 302. In such an embodiment, safety of the disinfecting device is improved.

Fig. 5a illustrates the light absorbing structures 304 comprising a plurality of evenly distributed wedge-shaped light converting elements 308. It is further conceivable that the light absorbing structures 404, shown in Fig. 5b, comprise a plurality of wedge-shaped elements 409, being arranged on the first surface 404' and the second surface 404" of the light absorbing structures 404. The light converting elements 408 are arranged on the wedge-shaped elements 409 in the form of a coating.

Although the present invention has been described with reference to various embodiments, those skilled in the art will recognize that changes may be made without departing from the scope of the claims.

It is intended that the detailed description be regarded as illustrative and that the appended claims the scope.

While the present invention has been illustrated in the appended drawings and the foregoing description, such illustration is to be considered illustrative or exemplifying and not restrictive; the present invention is not limited to the disclosed embodiments. Other variations to the disclosed embodiments can be understood and effected by those skilled in the art in practicing the claimed invention, from a study of the drawings, the disclosure, and the appended claims. In the appended claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A disinfecting device (1) comprising:
at least one light source (2) arranged for, in operation, to emit a first light having a first UV wavelength range;
at least one optical element (7) arranged to collimate at least a part of said first light emitted from said light source (2) towards at least one louver (3);
said at least one louver (3) comprising a plurality of light absorbing structures (4) having a first surface (4') and a second surface (4") being opposite to said first surface (4'), said light absorbing structure (4) further having a proximal end (5) being arranged adjacent to said at least one light source (2), and a distal end (6) being arranged opposite to said proximal end (5), said light absorbing structure (4) further having a longitudinal extension (L) between said proximal end (5) and said distal end (6), and a transversal extension (D) being substantially perpendicular to said longitudinal extension (L), said light absorbing structure (4) being arranged for absorbing a first portion of said first light;
**characterised in that**,
said plurality of light absorbing structures (4) comprises at least one light converting element (8) arranged for converting a second portion of said first light into a second light having a second wavelength range comprising longer wavelengths than said first UV wavelength range, and wherein a third portion of said first light is exiting said disinfection device (1),
wherein said light converting element (8) comprises a light converting material being arranged in a matrix being light transmissive to said first light.

2. The disinfecting device (1) according to claim 1, wherein said plurality of light absorbing structures (4) has an aspect ratio of at least 2.

3. The disinfecting device (1) according to any one of the preceding claims, wherein said at least one light converting element (8) is arranged on a portion of said first surface (4') or said second surface (4") of said light absorbing structure (4).

4. The disinfecting device (1) according to any one of claim 1 or 2, wherein said at least one light converting element (8) is arranged on a portion of said first surface (4') and said second surface (4") of said light absorbing structure (4).

5. The disinfecting device (1) according to claim 3 or 4, wherein the area of said portion of said first surface (4') and/or said second surface (4") comprising said at least one light converting element (8) is less than 30% of the total area of said first surface (4') and/or said second surface (4').

6. The disinfecting device (1) according to any one of claims 3-5, wherein said portion of said first surface (4') and/or said second surface (4") comprising said at least one light converting element (8) is arranged at said distal end (6) of said light absorbing structure (4).

7. The disinfecting device (1) according to any one of the preceding claims, wherein said plurality of light absorbing structures (4) comprising N light absorbing structures (4), wherein N is an integer being equal to or greater than five.

8. The disinfecting device (1) according to claim 7, wherein said plurality of light absorbing structures (4) is in the form of N substantially parallelly arranged plates.

9. The disinfecting device (101) according to any one of claims 7 or 8, wherein said at least one light converting element (8) is arranged on K light absorbing structures (4), wherein K is an integer having a value from one to N-1.

10. The disinfecting device (301) according to any one of the preceding claims, wherein said light absorbing structure (404) comprises at least one wedge-shaped element (409) being arranged on said first surface (404') and/or said second surface (404") of said light absorbing structure (404), and wherein said at least one wedge-shaped element (409) comprises said light converting element (408).

11. The disinfecting device (1) according to any one of the preceding claims, wherein said first UV wavelength range is from 100 nm to 280 nm, and wherein the second light has a major wavelength peak in the second wavelength range of 280 nm to 380 nm.

12. The disinfecting device (1) according to any one of the preceding claims, wherein said first UV wavelength range is from 100 nm to 280 nm, and wherein said second wavelength range is from 280 nm to 380 nm.

13. The disinfecting device (1) according to any one of the preceding claims 1 to 10, wherein said first UV wavelength range is from 100 nm to 280 nm, and wherein said second wavelength range is from 400 nm to 780 nm.

14. A luminaire comprising the disinfecting device (1) according to any one of the preceding claims and a mounting means for mounting said luminaire to a mounting surface.

15. A method for disinfection using the disinfection device (1) according to any one of claims 1 to 13 or using the luminaire according to claim 14.

## Patentansprüche

1. Desinfektionsvorrichtung (1), umfassend:
mindestens eine Lichtquelle (2), die angeordnet ist, um, im Betrieb, ein erstes Licht, das einen ersten UV-Wellenlängenbereich aufweist, auszusenden;
mindestens ein optisches Element (7), das angeordnet ist, um mindestens einen Teil des ersten Lichts, das von der Lichtquelle (2) ausgestrahlt wird, zu der mindestens einen Lamelle (3) hin zu kollimieren;
die mindestens eine Lamelle (3) umfassend eine Vielzahl von lichtabsorbierenden Strukturen (4), die eine erste Oberfläche (4') und eine zweiten Oberfläche (4"), die gegenüber der ersten Oberfläche (4') ist, aufweist, wobei die lichtabsorbierenden Struktur (4) ferner ein proximales Ende (5), das angrenzend an die mindestens eine Lichtquelle (2) angeordnet ist, und ein distales Ende (6) aufweist, das gegenüber dem proximalen Ende (5) angeordnet ist, wobei die lichtabsorbierenden Struktur (4) ferner eine Längsausdehnung (L) zwischen dem proximalen Ende (5) und dem distalen Ende (6) und eine Querausdehnung (D) aufweist, die im Wesentlichen senkrecht zu der Längsausdehnung (L) ist, wobei die lichtabsorbierenden Struktur (4) zum Absorbieren eines ersten Anteils des ersten Lichts angeordnet ist; **dadurch gekennzeichnet, dass** die Vielzahl von lichtabsorbierenden Strukturen (4) mindestens ein Lichtumwandlungselement (8) umfasst, das zum Umwandeln eines zweiten Anteils des ersten Lichts in ein zweites Licht, das einen zweiten Wellenlängenbereich aufweist, umfassend längere Wellenlängen als der erste UV-Wellenlängenbereich, angeordnet ist, und wobei ein dritter Anteil des ersten Lichts aus der Desinfektionsvorrichtung (1) austritt,
wobei das Lichtumwandlungselement (8) ein Lichtumwandlungsmaterial umfasst, das in einer Matrix angeordnet ist, die für das erste Licht durchlässig ist.

2. Desinfektionsvorrichtung (1) nach Anspruch 1, wobei die Vielzahl von lichtabsorbierenden Strukturen (4) ein Aspektverhältnis von mindestens 2 aufweist.

3. Desinfektionsvorrichtung (1) nach einem der vorstehenden Ansprüche, wobei das mindestens eine Lichtumwandlungselement (8) auf einem Anteil der ersten Oberfläche (4') oder der zweiten Oberfläche (4") der lichtabsorbierenden Struktur (4) angeordnet ist.

4. Desinfektionsvorrichtung (1) nach einem der Ansprüche 1 oder 2, wobei das mindestens eine Lichtumwandlungselement (8) auf einem Anteil der ersten Oberfläche (4') und der zweiten Oberfläche (4") der lichtabsorbierenden Struktur (4) angeordnet ist.

5. Desinfektionsvorrichtung (1) nach Anspruch 3 oder 4, wobei die Fläche des Anteils der ersten Oberfläche (4') und/oder der zweiten Oberfläche (4"), umfassend das mindestens eine Lichtumwandlungselement (8), weniger als 30 % der Gesamtfläche der ersten Oberfläche (4') und/oder der zweiten Oberfläche (4') beträgt.

6. Desinfektionsvorrichtung (1) nach einem der Ansprüche 3 bis 5, wobei der Anteil der ersten Oberfläche (4') und/oder der zweiten Oberfläche (4"), umfassend das mindestens eine Lichtumwandlungselement (8), an dem distalen Ende (6) der lichtabsorbierenden Struktur (4) angeordnet ist.

7. Desinfektionsvorrichtung (1) nach einem der vorstehenden Ansprüche, wobei die Vielzahl von lichtabsorbierenden Strukturen (4) N lichtabsorbierende Strukturen (4) umfasst, wobei N eine ganze Zahl ist, die gleich oder größer als fünf ist.

8. Desinfektionsvorrichtung (1) nach Anspruch 7, wobei die Vielzahl der lichtabsorbierenden Strukturen (4) in der Form von N im Wesentlichen parallel angeordneten Platten vorliegt.

9. Desinfektionsvorrichtung (101) nach einem der Ansprüche 7 oder 8, wobei das mindestens eine Lichtumwandlungselement (8) auf K lichtabsorbierenden Strukturen (4) angeordnet ist, wobei K eine ganze Zahl ist, die einen Wert von eins bis N-1 aufweist.

10. Desinfektionsvorrichtung (301) nach einem der vorstehenden Ansprüche, wobei die lichtabsorbierende Struktur (404) mindestens ein keilförmiges Element (409) umfasst, das auf der ersten Oberfläche (404') und/oder der zweiten Oberfläche (404") der lichtabsorbierenden Struktur (404) angeordnet ist, und wobei das mindestens eine keilförmige Element (409) das Lichtumwandlungselement (408) umfasst.

11. Desinfektionsvorrichtung (1) nach einem der vorstehenden Ansprüche, wobei der erste UV-Wellenlängenbereich zwischen 100 nm und 280 nm liegt und wobei das zweite Licht eine Hauptwellenlängenspitze in dem zweiten Wellenlängenbereich von 280 nm bis 380 nm aufweist.

12. Desinfektionsvorrichtung (1) nach einem der vorstehenden Ansprüche, wobei der erste UV-Wellenlängenbereich zwischen 100 nm und 280 nm liegt und wobei der zweite Wellenlängenbereich zwischen 280 nm und 380 nm liegt.

13. Desinfektionsvorrichtung (1) nach einem der vorstehenden Ansprüche 1 bis 10, wobei der erste UV-Wellenlängenbereich zwischen 100 nm und 280 nm liegt und wobei der zweite Wellenlängenbereich zwischen 400 nm und 780 nm liegt.

14. Leuchte, umfassend die Desinfektionsvorrichtung (1) nach einem der vorstehenden Ansprüche und ein Befestigungsmittel zum Befestigen der Leuchte an einer Befestigungsoberfläche.

15. Verfahren für eine Desinfektion unter Verwendung der Desinfektionsvorrichtung (1) nach einem der Ansprüche 1 bis 13 oder unter Verwendung der Leuchte nach Anspruch 14.

## Revendications

1. Dispositif de désinfection (1) comprenant :
au moins une source de lumière (2) agencée pour, en fonctionnement, émettre une première lumière ayant une première plage de longueur d'onde UV ;
au moins un élément optique (7) agencé pour collimater au moins une partie de ladite première lumière émise par ladite source de lumière (2) en direction d'au moins une persienne (3) ;
ladite au moins une persienne (3) comprenant une pluralité de structures d'absorption de lumière (4) ayant une première surface (4') et une seconde surface (4") étant opposée à ladite première surface (4'), ladite structure d'absorption de lumière (4) ayant en outre une extrémité proximale (5) étant agencée adjacente à ladite au moins une source de lumière (2), et une extrémité distale (6) étant agencée opposée à ladite extrémité proximale (5), ladite structure d'absorption de lumière (4) ayant en outre une extension longitudinale (L) entre ladite extrémité proximale (5) et ladite extrémité distale (6), et une extension transversale (D) étant sensiblement perpendiculaire à ladite extension longitudinale (L), ladite structure d'absorption de lumière (4) étant agencée pour absorber une première partie de ladite première lumière ; **caractérisé en ce que,** ladite pluralité de structure d'absorption de lumière (4) comprend au moins un élément de conversion de lumière (8) agencé pour convertir une deuxième partie de ladite première lumière en une seconde lumière ayant une seconde plage de longueur d'onde comprenant des longueurs d'onde plus longues que ladite première plage de longueur d'onde UV, et dans lequel une troisième partie de ladite première lumière sort dudit dispositif de désinfection (1),
dans lequel ledit élément de conversion de lumière (8) comprend un matériau de conversion de lumière étant agencé dans une matrice transmettant la lumière pour ladite première lumière.

2. Dispositif de désinfection (1) selon la revendication 1, dans lequel ladite pluralité de structures d'absorption de lumière (4) a un rapport d'aspect d'au moins 2.

3. Dispositif de désinfection (1) selon l'une quelconque des revendications précédentes, dans lequel ledit au moins un élément de conversion de lumière (8) est agencé sur une partie de ladite première surface (4') ou de ladite seconde surface (4") de ladite structure d'absorption de lumière (4).

4. Dispositif de désinfection (1) selon l'une quelconque des revendications 1 ou 2, dans lequel ledit au moins un élément de conversion de lumière (8) est agencé sur une partie de ladite première surface (4') et de ladite seconde surface (4") de ladite structure d'absorption de lumière (4).

5. Dispositif de désinfection (1) selon la revendication 3 ou 4, dans lequel l'aire de ladite partie de ladite première surface (4') et/ou de ladite seconde surface (4") comprenant ledit au moins un élément de conversion de lumière (8) est inférieure à 30 % de l'aire totale de ladite première surface (4') et/ou de ladite seconde surface (4').

6. Dispositif de désinfection (1) selon l'une quelconque des revendications 3 à 5, dans lequel ladite partie de ladite première surface (4') et/ou de ladite seconde surface (4") comprenant ledit au moins un élément de conversion de lumière (8) est agencée au niveau de ladite extrémité distale (6) de ladite structure d'absorption de lumière (4).

7. Dispositif de désinfection (1) selon l'une quelconque des revendications précédentes, dans lequel ladite pluralité de structures d'absorption de lumière (4) comprend N structures d'absorption de lumière (4), dans lequel N est un nombre entier étant égal ou supérieur à cinq.

8. Dispositif de désinfection (1) selon la revendication 7, dans lequel ladite pluralité de structures d'absorption de lumière (4) est sous la forme de N plaques agencées de façon sensiblement parallèle.

9. Dispositif de désinfection (101) selon l'une quelconque des revendications 7 ou 8, dans lequel ledit au moins un élément de conversion de lumière (8) est agencé sur K structures d'absorption de lumière (4), dans lequel K est un nombre entier ayant une valeur allant de un à N-1.

10. Dispositif de désinfection (301) selon l'une quelconque des revendications précédentes, dans lequel ladite structure d'absorption de lumière (404) comprend au moins un élément en forme de coin (409) étant agencé sur ladite première surface (404') et/ou ladite seconde surface (404") de ladite structure d'absorption de lumière (404), et dans lequel ledit au moins un élément en forme de coin (409) comprend ledit élément de conversion de lumière (408).

11. Dispositif de désinfection (1) selon l'une quelconque des revendications précédentes, dans lequel ladite première plage de longueur d'onde UV va de 100 nm à 280 nm, et dans lequel la seconde lumière a un pic majeur de longueur d'onde dans la seconde plage de longueur d'onde de 280 nm à 380 nm.

12. Dispositif de désinfection (1) selon l'une quelconque des revendications précédentes, dans lequel ladite première plage de longueur d'onde UV va de 100 nm à 280 nm, et dans lequel ladite seconde plage de longueur d'onde va de 280 nm à 380 nm.

13. Dispositif de désinfection (1) selon l'une quelconque des revendications 1 à 10 précédentes dans lequel ladite première plage de longueur d'onde UV va de 100 nm à 280 nm, et dans lequel ladite seconde plage de longueur d'onde va de 400 nm à 780 nm.

14. Luminaire comprenant le dispositif de désinfection (1) selon l'une quelconque des revendications précédentes et un moyen de montage permettant de monter ledit luminaire sur une surface de montage.

15. Procédé permettant la désinfection à l'aide du dispositif de désinfection (1) selon l'une quelconque des revendications 1 à 13 ou à l'aide du luminaire selon la revendication 14.
